# EUROPEAN PATENT SPECIFICATION

(11) **EP 1 779 111 B1**
(45) Date of publication and mention of the grant of the patent: **07.07.2010**
(21) Application number: 05758927.7
(22) Date of filing: 15.07.2005
(51) Int. Cl.: G01N 33/543, B01L 3/00

(54) **GRADING OF IMMUNE RESPONSES**
ABSTUFUNG VON IMMUNANTWORTEN
STIMULATION DES RÉPONSES IMMUNITAIRES

(30) Priority: 16.07.2004 SE 0401888; 16.07.2004 US 588712 P
(43) Date of publication of application: 02.05.2007
(73) Proprietor: GYROS PATENT AB, 751 83 Uppsala (SE)
(72) Inventor: INGANÄS, Mats, S-757 55 Uppsala (SE)
(74) Representative: Franks, Barry Gerard
(86) International application number: PCT/SE2005/001153
(87) International publication number: WO 2006/009505

(56) References cited:
- WO-A1-02/075312
- LAENGE K ET AL: "Flow injection immunotitration: Extended working range for inhibition type immunodetection" 15 November 1999 (1999-11-15), ANALYTICA CHIMICA ACTA, VOL. 399, NR. 3, PAGE(S) 275-286 , XP002484630 ISSN: 0003-2670 * the whole document *
- SEM DANIEL S ET AL: "Antibody affinities and relative titers in polyclonal populations: Surface plasmon resonance analysis of anti-DNA antibodies" 1 December 1999 (1999-12-01), ARCHIVES OF BIOCHEMISTRY AND BIOPHYSICS, VOL. 372, NR. 1, PAGE(S) 62-68 , XP002484631 ISSN: 0003-9861 * the whole document *
- NERI D ET AL: "Biophysical methods for the determination of antibody-antigen affinities" 1 December 1996 (1996-12-01), TRENDS IN BIOTECHNOLOGY, ELSEVIER PUBLICATIONS, CAMBRIDGE, GB, PAGE(S) 465 - 470 , XP004071037 ISSN: 0167-7799 * the whole document *
- HELG A ET AL: "Comparison of analytical methods for the evaluation of antibody responses against epitopes of polymorphic protein antigens" 1 May 2003 (2003-05-01), JOURNAL OF IMMUNOLOGICAL METHODS, ELSEVIER SCIENCE PUBLISHERS B.V.,AMSTERDAM, NL, PAGE(S) 19 - 31 , XP004422638 ISSN: 0022-1759 * the whole document *
- PIERSON L. ET AL: 'An automated method for determination of antibody affinity distribution funtions with nanogram quantities' JOURNAL OF IMMUNOLOGY METHODS vol. 211, 1998, pages 97 - 109, XP004120569
- FERREIRA M.U. ET AL: 'The assesment of antibody affinity distribution by thiocyanate elution: a simple dose-response approach' JOURNAL OF IMMUNOLOGICAL METHODS vol. 187, 1995, pages 297 - 305, XP004020960
- PROLL G. ET AL: 'Monitoring an antibody affinity chromatography with a label-free optical biosensor technique' JOURNAL OF IMMUNOLOGICAL METHODS vol. 292, 2004, pages 35 - 42, XP004550498

## Description

### FIELD OF THE INVENTION

The present invention concerns a method for qualitatively grading a polyclonal antibody response provoked by an antigenic agent. The grading is typically with respect to binding specificity of antibodies of the response towards a structure (binding structure = BS) that is related to the antigenic agent, and typically includes an estimation of the presence and/or absence of high affinity antibodies that are capable of binding to the binding structure. The grading typically also comprises determination of Ig-class and/or subclass of one or more the antibodies of the response, among others.

The term "Ig" refers to immunoglobulin and encompasses the corresponding group of substances or proteins in other vertebrates.

### BACKGROUND TECHNOLOGY AND OBJECTS OF THE INVENTION

Upon infection or exposure to exogenous antigens as well as upon attempts to actively induce an immune response for vaccination or for preparing antibodies to be used as reactants in assays, capturing, drugs etc, a humoral immune response is evoked and developed over significant periods of time. A full-fledged immune response for protection against exogenous organisms may take several months to develop in a host, typically a vertebrate such as a mammal, an avian etc. An immune response resulting in antibodies to be used as reactants in immune assays and/or as immobilized or immobilizable capture reactants imposes other quality criteria and may need to be interrupted at a certain period of time depending on the particular use of the antibodies. The antibody diversity created in an immune response over time may shift between immunoglobulin (Ig) classes/subclasses but may also evolve on a molecular level, i.e. a process of refining the immune response in terms of epitope specificity and affinity, i.e. binding strength. The latter process is called "affinity maturation". A humoral immune response is polyclonal in the sense that it comprises a spectrum of antibodies originating from different antibody-producing cells and differing with respect to epitope specificity, Ig-class/subclass, affinity etc.

Currently there are no analytical systems that actively and effectively can differentiate humoral immune responses according to their content of low and/or high affinity antibodies.

Assay conditions may sometimes be such that high affinity antibodies are preferentially selected for, but in most cases large sample volumes, long diffusion distances and long incubation times will prevent selection of reactive antibodies according to affinity. Typical assays often measure antibodies of limited significance for an intended use, for instance as a biological marker (i.e. a diagnostic marker for a disease, an infection and the like), as a reactant in an immune assay, as an immobilized or immobilizable capture reactant etc. In diagnostic antigen specific antibody assays clinically less important antibodies may be "included" in the assay result obscuring clinically important antibodies and affecting diagnostic accuracy negatively. Most assays dedicated of assaying presence of antibodies have difficulties in differentiating negative responses from weak positive ones.

WO 02075312, WO 03018198, WO04083108 and WO 04083109 (all Gyros AB) describe among others methods and microfluidic devices that can be adapted to the present innovative method.

The objects of the invention are to overcome the problems discussed above.

### DRAWINGS

**Figure 1** illustrates a suitable subset of microchannel structures on a microfluidic device.
**Figure 2** illustrates schematically the affinity complex formed on the solid phase

### THE INVENTION

The present inventor has recognized that the above-mentioned drawbacks of antigen specific antibody assays and grading of humoral immune responses can be overcome by utilizing an antigen-specific antibody assay, which comprises the steps of:
(i) providing a reaction microcavity having an inlet end and an outlet end and containing a solid phase that exposes an immobilized binding structure (BS) that is capable of affinity binding antibodies (Ab) of the response,
(ii) flowing a liquid sample containing antibodies of the response in the direction from the inlet end to the outlet end through the solid phase,
(iii) determining the distribution of antibodies, which are captured during step (ii), along the flow direction of the solid phase, and
(iv) grading the response based on the distribution determined in step (iii).

### STEP (i): REACTION MICROCAVITY (104a-h), THE IMMOBILIZED BINDING STRUCTURE, AND THE SOLID PHASE MATERIAL.

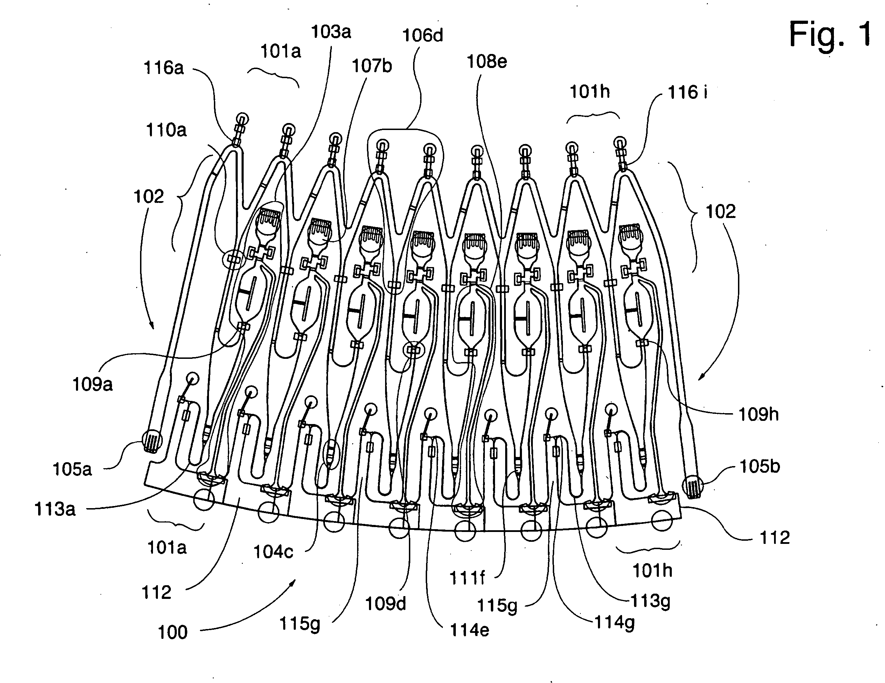

The reaction microcavity **(104a-h)** is typically located between an inlet port and an outlet port of a microchannel structure **(101a-h)** of a microfluidic device. The microfluidic device as such and other parts of a microchannel structure **(101a-h)** is presented in a separate section below. See also figure 1.

The reaction microcavity **(104a-h)** is defined as the part of the microchannel structure **(101ah)** where the solid phase carrying the binding structure is present. The microcavity **(104a-h)** thus has an upstream/inlet end and a downstream/outlet end, each of which coincides with the upstream/inlet end and the downstream/outlet end, respectively, of the solid phase. The microcavity may be part of a larger chamber comprising a different solid, for instance being devoid of a binding stricture or exposing binding structures having othe specificities. Each such soli phase defines other reaction microcavities.

The reaction microcavity **(104a-h)** used in the present invention typically also functions as a detection microcavity.

The reaction microcavity **(104a-h)** is typically a straight microconduit or may or may not be widening and/or narrowing in the flow direction. A reaction microcavity may in addition also exhibit a curvature, for instance comprising one or more bents such as being S-shaped, or be rounded, drop-shaped etc.

The reaction microcavity **(104a-h)** typically has at least one cross-sectional dimension that is ≤ 1,000 µm, such as ≤ 500 µm or ≤ 200 µm (depth and/or width). The smallest cross-sectional dimension is typically ≥ 5 µm such as ≥ 25 µm or ≥ 50 µm. The total volume of the reaction microcavity is typically in the nl-range, such as ≤ 5,000 nl, such as 1,000 nl or ≤ 500 nl ≤ 100 nl or ≤ 50 nl or ≤ 25 nl.

The solid phase may be in the form of porous bed, i.e. in the form of a porous monolithic bed or a bed of packed particles that may be porous or non-porous. Alternatively, the solid phase may be an inner wall of the reaction microcavity.

A monolithic bed may be in the form of a porous membrane or a porous plug.

The term "porous particles" have the same meaning as in WO 02075312 (Gyros AB).

Suitable particles are spherical or spheroidal (beaded), or non-spherical. Appropriate mean diameters for particles are typically found in the interval of 1-100 µm with preference for mean diameters that are ≥ 5 µm, such as ≥ 10 µm or ≥ 15 µm and/or ≤ 50 µm. Also smaller particles can be used, for instance with mean diameters down to 0.1 µm. The design of the outlet end **(111a-h)** of the reaction microcavity **(104a-h)** and the particles should match each other so that the particles can be retained in the reaction microcavity **(104a-h).** Certain kinds of particles, in particular particles of colloidal dimension, may agglomerate. In these cases the size of the agglomerate should be in the intervals given even if the agglomerating particles as such are smaller. See for instance WO 02075312 (Gyros AB). Diameters refer to the "hydrodynamic" diameters.

Particles to be used may be monodisperse (monosized) or polydisperse (polysized) in the same meaning as in WO 02075312 (Gyros AB).

The solid phase material may or may not be transparent.

The base material of a solid phase may be made of inorganic and/or organic material. Typical inorganic materials comprise glass and typical organic materials comprise organic polymers. Polymeric materials comprise inorganic polymers, such as glass and silicone rubber, and organic polymers that may be of synthetic or biological origin (biopolymers). The term biopolymer includes semi-synthetic polymers in which there is a polymer backbone derived from a native biopolymer. Typical synthetic organic polymers are cross-linked and are often obtained by the polymerisation of monomers comprising polymerisable carbon-carbon double bonds. Examples of suitable monomers are hydroxy alkyl acrylates, for instance 2-hydroxyalkyl acrylates, and corresponding methacrylates, acryl amides and methacrylamides, vinyl and styryl ethers, alkene substituted polyhydroxy polymers, styrene, etc. Typical biopolymers may or may not be cross-linked. In most cases they exhibit carbohydrate structure, e.g. agarose, dextran, starch etc.

The term "hydrophilic" in the context of a porous bed contemplates a sufficient wettability of the surfaces of the pores for water to be spread by capillarity all throughout the bed when in contact with excess water (absorption). The expression also means that the inner surfaces of the bed that is in contact with an aqueous liquid medium during the capture of antibodies of the response (immune absorption) shall expose a plurality of polar functional groups which each has a heteroatom selected amongst oxygen and nitrogen, for instance. Appropriate functional groups can be selected amongst hydroxy groups, ethylene oxide groups (-X-[CH₂CH₂O-]ₙ where n is an integer > 1 and X is nitrogen or oxygen), amino groups, amide groups, ester groups, carboxy groups, sulphone groups etc, with preference for those groups that are essentially uncharged independent of pH, for instance within the interval of 2-12. For solid phase materials in particle form this means that at least the outer surfaces of the particles have to exhibit polar functional groups. The hydrophilic functional groups may be present on or be a part of so called extender arms (tentacles). Similar material, wettabilities and functional groups etc also apply to solid phases in the form of inner walls.

If the base material of a solid phase material is hydrophobic or not sufficiently hydrophilic, e.g. is based on a styrene (co)polymer, the surfaces that are to be in contact with an aqueous liquid may be hydrophilized. Typical coating protocols comprise coating with a compound or mixture of compounds exhibiting polar functional groups of the same type as discussed above, treatment by an oxygen plasma etc.

In certain variants there is a risk that the sample used contains aggregated immunoglobulins that may disturb measurement of antigen specific antibodies on the solid phase (step (iii). In the case the solid phase is a porous bed it may then be advantageous to select pore dimensions of the bed such that aggregated immunoglobulins can pass through without being physically trapped within the bed to any significant degree. Alternatively, one can arrange for filtering means upstream the bed, for instance by placing a porous bed lacking the binding structure (dummy bed) in front of the solid phase exhibiting the binding structure. This extra porous bed and/or filter may be abutted to the inlet of the solid phase/reaction microcavity **(104a-h)** or physically separated therefrom. See further PCT/SE2005/000114 (Gyros AB).
The term "a binding structure" (BS) includes one or more binding structures in the form of epitopes, antigens, haptens and the like which are capable of affinity binding/reacting to/with antibodies of the immune response concerned. The binding structure is thus related to the antigenic agent that has provoked the immune response in the sense that the binding structure and this agent have epitopes that are reacting with the same antibody in the response (common epitopes). Epitopes of this kind may or may not be structurally identical. It is imperative that the binding occurs via an antigen/hapten-binding part of the antibody.

The binding structure (BS) on the solid phase should be present in excess compared to the amount of antibodies of the response that will actually bind specifically to the solid phase during step (iii). The excess typically is more than 5-fold such as more than 10-fold or more than 25-fold or more than 50 fold or more than 100-fold, typically measured as free binding structures available after capturing.

A humoral immune response can in principle be provoked against any structure that is foreign to a particular host. This means that the immobilized binding structure may be selected amongst a large variety of structures provided they are related to the agent provoking the immune response studied. Thus the binding structure may exhibit
a) amino acid structure including peptide structure such as poly and oligopeptide structure, including mimetics and chemically modified forms of these structures etc;
b) carbohydrate structure, including mimetics and chemically modified forms of these structures, etc;
c) nucleotide structure including nucleic acid structure, including mimetics and chemically modified variants of these structures, etc;
d) lipid structure such as steroid structure, triglyceride structure, etc, including mimetics and chemically modified forms of these structures;
e) etc
A number of other structures and substances are also included such as haptenic/antigenic structures that may be present in infectious agents (bacteria, algae, fungi, viruses, prions, moulds, parasites etc), drugs, autoantigens, allergens, synthetic or native immunogens that are capable of provoking humoral immune responses for the manufacture of an antigen specific antibody reagent or drug.

The techniques for the introduction of the binding structure on the solid phase is typically according to one or both of two main routes:
a) immobilization of a reactant comprising the binding structure and/or the reactant to the solid phase, and
b) building the structure stepwise on the solid phase (solid phase synthesis of an immobilised reactant exhibiting the binding structure).

Both routes are commonly known in the field. The linkage to the solid phase material may thus be via covalent bonds, affinity bonds (for instance biospecific affinity bonds), physical adsorption, electrostatic bonds etc.

Covalent immobilization is typically carried out by reacting mutually reactive functional groups on the desired reactant and the solid phase, respectively, with each other to the formation of a covalent bond attaching the binding structure to the solid phase. In the case the solid phase and reactant do not contain this kind of groups, either one or both of them are normally activated to exhibit suitable functional groups. The functional group that can be used on the desired reactant is typically selected amongst electrophilic and nucleophilic groups. The exact choice depends on whether or not the functional group on the solid phase is nucleophilic or electrophilic, respectively. Examples of functional groups that may be used are amino groups and other groups comprising substituted or unsubstituted -NH₂, carboxy groups (-COOH/-COO⁻), hydroxy groups, thiol groups, disulfide groups, carbonyl groups (keto, aldehyde), carbon-carbon double and triple bonds, haloalkyl groups etc. Free radical reactions may also be used for the covalent introduction of binding structures on the solid phase.

Immobilization via affinity bonds may utilize an immobilizing affinity pair in which one of the members (immobilized ligand or L) is firmly attached to the solid phase material, for instance covalently. The other member (immobilizing binder, B) of the pair is used as a conjugate (immobilizing conjugate) comprising binder B and the binding structure. Examples of immobilizing affinity pairs are a) streptavidin/avidin/neutravidin and a biotinylated reactant (or vice versa), b) antibody and a haptenylated reactant (or vice versa), c) an IMAC group and an amino acid sequence containing histidyl and/or cysteinyl and/or phosphorylated residues (i.e. an IMAC motif) linked to or being a part of a reactant, etc.

The term "conjugate" primarily refers to covalent conjugates, such as chemical conjugates and recombinantly produced conjugates (where both the moieties and the linker between them have peptide structure). The term also includes so-called native conjugates, i.e. affinity reactants which each exhibits two binding sites that are spaced apart from each other with affinity directed towards two different molecular entities. Native conjugates thus includes antigens which each has physically separated antigenic determinants, antibodies which each comprises species and/or class-specific determinants in one part of the molecule and an antigen/hapten-binding site on another part.

It is believed that it is advantageous that the immobilized ligand L has two or more binding sites for the immobilizing binder B, and/or the immobilizing binder B has one, two or more binding sites for the ligand L (or vice versa).

Preferred immobilizing affinity pairs (L and B) typically have affinity constants (K_{L--B} = [L][B]/[L--B]) that are at most equal to the corresponding affinity constant for streptavidin and biotin, or ≤ 10¹ times or ≤ 10² times or ≤ 10³ times larger than this latter affinity constant. This typically will mean affinity constants that roughly are ≤ 10⁻¹³ mole/l, ≤ 10⁻¹² mole/l, ≤ 10⁻¹¹ mole/l and ≤ 10⁻¹⁰ mole/1, respectively. The preference is to select L and B amongst biotin-binding compounds and streptavidin-binding compounds, respectively, or vice versa.

The affinity constants discussed above refer to values obtained by a biosensor (surface plasmon resonance) from Biacore (Uppsala, Sweden), i.e. with the ligand L immobilized to a dextran-coated gold surface.

Immobilization by physical adsorption is well known in the field and mostly utilizes hydrophobic groups on both the reactant to be immobilized and the solid phase. Immobilization by electrostatic bonds is also well known in the field and utilizes reactants comprising a plurality of charges combined with solid phase surfaces exhibiting a plurality of the opposite charges.

### STEP (ii): FLOW CONDITIONS AND CAPTURING

The term "flowing" in step (ii) means that the antibodies of the humoral immune response is presented to the immobilized binding structure under flow conditions, with particular emphasis for flow rates favouring non-diffusion limiting conditions for reaction. In preferred variants the flow rate is selected to provide non-diffusion limiting conditions for reaction between at least a part of the antibodies in the sample and the binding structure. These kinds of conditions result in an enrichment (peak) of captured antibodies at the section of the solid phase next to the inlet end. The antibodies captured in the peak are typically of higher affinity than those that are captured downstream the peak or have passed through the solid phase without being captured.

Flow conditions, in particular favoring capturing of high affinity antibodies as discussed above will also favor detection of high affinity antibodies, i.e. antibodies having affinity constants (K_{BS-Ab} = [BS--Ab]/ [BS] [Ab]) ≥ 10⁸ 1/mole, such as ≥ 10⁹ l/mole or ≥ 10¹⁰ 1/mole or ≥ 10¹² 1/mole. BS refers to the binding structure that is relevant to a particular antibody, Ab to the particular antibody, and BS--Ab to the affinity complex between the antibody Ab and the binding structure BS. These affinity constants refer to values obtained by a biosensor (surface plasmon reference) from Biacore (Uppsala, Sweden), i.e. with the binding structure (BS) immobilized to a dextran-coated gold surface.

The appropriate flow rate through the porous bed depends on a number of factors:
a) the immobilized reactant (BS);
b) the kind of antibodies in the response (species in which the response have been obtained, IgG-class, actual affinity constant of the antibodies of interest;
c) the dimensions of the reaction microcavity (volume, length etc),
d) the kind of solid phase (the solid phase material, porosity, bed or coated inner wall etc); and
e) etc.
Typically the flow rate should give a residence time of ≥ 0.010 seconds such as ≥ 0.050 sec or ≥ 0.1 sec with an upper limit that typically is below 2 hours such as below 1 hour. Illustrative flow rates are within 0.001-10,000 nl/sec, such as 0.01-1,000 nl/sec or 0.01-100 nl/sec or 0.1 - 10 nl/sec. These flow rate intervals may primarily be useful for solid phase volumes in the range of 1-1,000 nl, such as 1-200 nl or 1-50 nl or 1-25 nl. Residence time refers to the time it takes for a liquid aliquot to pass the solid phase in the reaction cavity. Optimization typically will require experimental testing on each particular system.

The liquid flow through the solid phase can be driven by in principle any kind of forces, for instance by electrokinetically or non-electrokinetically created forces. See under the heading "General about the microfluidic device". Centrifugal force created by spinning a microfluidic device which comprises one, two or more of the above-mention reaction microcavities **(104ah)** in separate microchannel structures are particularly advantageous since this kind of force easily provides for flow control that is common for all of the reaction microcavities and microchannel structures. By arranging equal microchannel structures symmetrically around the spin axis it is easily to obtain the same flow rate through each reaction microcavity. Further advantages with centrifugal force are that flow rate easily can be varied, e.g. by changing the spin speed or by having microchannel structures at different distance from the spin axis, etc. Compare step (iv) below. Other liquid flow creating arrangements that provide for common flow control are also potentially advantageous. Common flow control as herein contemplated is discussed in detail in WO 02075312 (Gyros AB).

### STEP (iii). DETERMINATION OF DISTRIBUTION OF ANTIBODIES IN THE SOLID PHASE.

The antibody captured on the solid phase in step (ii) may be detected by methods well known in the field. A preferred variant utilizes an analytically detectable affinity reactant that is a conjugate between a signal-generating label (moiety 1) and an affinity reactant (moiety 2) that is capable of affinity binding to the antibodies of the response that have been captured during step (ii). The affinity binding of this labeled conjugate to a captured antibody may be directly between moiety 2 and a class, subclass or species specific determinant on the antibody. Alternatively the binding may be indirectly via one or more intermediary affinity reactants (conjugates), each of which has a dual binding specificity. An intermediary reactant may inherently comprise a dual specificity, for instance be an antibody. Alternatively an intermediary reactant may be a synthetic conjugate, for instance comprising one part (moiety 2') that is directed towards a class, subclass or species specific determinant on the captured antibody and a second part (moiety 1') that is directed towards moiety 2 of the labelled conjugate.

A conjugate - either it be native or synthetic- used in the invention for affinity binding directly to a captured antibody typically comprises anti-antibody binding activity towards species specific and/or class and/or subclass specific determinants on the captured antibody. Such conjugates will henceforth be included in the term anti-antibody with emphasis of specificity for the main mammalian Ig-classes, i.e. anti-IgA, anti-IgD, anti-IgE, anti-IgG, and anti-IgM antibodies and the corresponding classes in other vertebrates. There is also a preferred use of anti-antibodies specific for subclasses, i.e. for human subclasses means anti-IgA1, anti-IgA2, anti-IgG1, anti-IgG2, anti-IgG3, and anti-IgG4 antibodies. Anti-species antibodies are selected according origin of the sample to be studied and include anti-human, anti-rabbit, antimouse, anti-rat, anti-guinea pig, anti-horse, anti-cow, anti-pig and anti-hen antibodies, among others.

Typical examples of moiety 1' (affinity tags) are biotin, hapten, metal chelate (IMAC group) etc with their affinity counterparts as given elsewhere in this text.

Typical signal-generating labels include radiation-generating labels, such as enzymes, chromogens, fluorogens, fluorophors, chemiluminescent groups, radioactive groups etc. A key part of the invention is to measure the distribution of the captured antibody in the reaction microcavity/solid phase. This means that a signal-generating label must give rise to a signal that can be measured at the same position within the solid phase as where the labelled conjugate creating the signal is binding to a captured antibody. For enzymes as labels this means that the product formed should be immediately fixated (immobilized) to the solid phase where the product is formed. No significant transport by diffusion or convection should be permitted. See for instance WO 04106926 (Gyros AB). This also means that radiation-emitting labels many times are preferred, e.g. fluorophors, fluorogens, chromogens, chemiluminscent labels etc, both as labels on affinity reactants and as labels in the immobilized product formed when an enzyme is used as a label on an affinity reactant.

A signal-generating label may be combined with a second signal-generating label in the above-mentioned complex. This second label is selected such that the two labels together give the appropriate signal when the complex is formed or dissociated. This variant may be illustrated with scintillation proximity assays (SPA) in which a soluble affinity reactant, which is labeled with tritium, is used together with a solid phase comprising a scintillation substance. When the labeled reactant becomes incorporated into a complex bound to this kind of solid phase a signal will appear. The principle with interacting labels may also be illustrated with pairs of fluorophores that may be identical or different and with fluorescence-quencher pairs.

In still another variant the immobilized binding structure (BS) is associated with a signal-generating label for which the signal is changed when an antibody of the humoral immune response is captured.

Another potential variant utilizes a detectable reactant that is capable of affinity binding to excessive binding structures on the solid phase after step (ii), i.e. structures that remain unoccupied after step (ii).

Still another potential variant comprises preincubation of the sample with an analytically detectable affinity reactant that is capable of reacting with species specific or class/subclass specific determinants on antibodies of the humoral immune response. The sample is subjected to step (ii) after the pre-incubation.

The measurement of the signal from the solid phase typically results in a set of data (pixels) that can be represented as an image of the solid phase (detection area) as viewed through a detection window/search area of/in the surface of the microfluidic device. See WO 03025548, US 20030156763 and WO 03056517 (all Gyros AB). Many times it is of advantage to reduce noise in the image/data set, for instance by performing at least one of the operations: a) reducing background disturbances, b) reducing peak disturbances, c) locating a detection area within a search area, d) (re)moving binary artifacts, e) removing unwanted areas of a detection area, and f) applying default detection area to noisy images. See WO 03056517 and US SN 10/331,399 (all Gyros AB).

The data set is then used for determination of the distribution of the antibody captured in step (ii).

In assay protocols where the signal from the detectable entity directly or indirectly derives from the affinity complex formed during the capturing step (ii) (complex comprising BS-Ab), an increase in the measured signal in a pixel will represent an increase in the amount of of captured antibodies in the underlying part of the solid phase. In protocol variants where the signal derives from excessive binding structures (BS) (see above), a decrease in the measured signal in a pixel will represent an increase in the amount of captured antibodies in the underlying part of the solid phase.

The distribution may then be measured as the relative amount of captured immune response antibodies in the section of the column that is next to the inlet end of the solid phase containing binding structure BS (upper section), for instance encompassing ≤ 75 %, with preference for ≤ 50 % or ≤ 30 % of (a) the length of the solid phase/reaction microcavity, or (b) the part of the solid phase/reaction microcavity from which signal over the tresh-hold can be measured. This relative amount may be called "peak".

### STEP (iv): GRADING OF THE RESPONSE

The relative amount of captured antibodies measured in an upper section (peak) as defined above is taken as an indication of the amount of high affinity anti-BS antibodies on the solid phase and in the sample. This relative amount may be ≥ 50 % such as ≥ 60 % or ≥ 70 % or ≥ 80 % for each of the intervals mentioned for the lengths of sections. A higher amount in an upper section typically will represent an elevated level (or higher proportion) of high affinity anti-BS antibodies than a lower amount in the same section. The same amount in a shorter upper section typically will represent antibodies of higher affinities. A narrower peak (in the flow-direction) will be indicative of higher affinities than a broader peak. The narrowness of a peak can be measured as a decrease in the ratio: peak height to peak width at a predetermined part of the peak height, e.g. at the half of the peak height.

Data treatment for the determination of the distribution in step (iii) and the grading in step (iv) is typically performed by the aid of a suitable data program, for instance based on WO 03025548, US 20030156763 and WO 03056517 (all Gyros AB).

In many cases a rough estimate of the distribution can be carried out by visual inspection of the reaction microcavity, possibly while enlarging the view of it. This kind of simplified estimation of high affinity antibodies may also be used as an alternative in steps (iii) and (iv).

The inventive method is typically part of a method for quantitatively measuring antigen-specific antibodies of an immune response in a sample containing such antibodies, i.e. a quantitative measurement of the amount of these antibodies is determined from the signals collected during steps (i)-(iii), possibly by the use of reference samples containing known amounts or levels. See WO 03025548, US 20030156763 and WO 03056517 (all Gyros AB).

Low affinity anti-BS antibodies will have a tendency to escape through the solid phase without being bound and/or detected. In certain variants of the invention it may be of interest to include also an estimation of immune response anti-BS antibodies of lower affinity in the grading. This can be accomplished by repeating steps (i) - (iv) with a slower flow rate during the affinity capture step (ii). Alternatively a conventional immunoassay may be performed during static conditions for a period of time allowing for the capturing reaction to go to equilibrium. In the first alternative the chances for capturing anti-BS antibodies of lower affinity will increase, i.e. the peak will be more distinct. In the second alternative both low and high affinity anti-BS antibodies will be measured and a comparison with a run according to steps (i)-(iv) potentially will give a better estimation of the level of low affinity anti-BS antibodies in the sample since the comparison will account for low affinity anti-BS antibodies that have passed through the reaction microcavity without being detected.

The inventive method also comprises that a number of different experiments according to the invention is carried out on separate aliquots, for instance from the same mother sample. Mother sample in this context includes essentially identical samples collected from the same individual at essentially the same time. These experiments (including the first experiment) may comprise:
1) A difference with respect to the specificity of the affinity reactant used for detecting the captured antibodies. Thus by using affinity reactants that are specific for different class specific and/or subclass specific determinants, antibodies of the corresponding class and/or subclass can be measured. This can be used for determining the distribution of classes and/or subclasses of antigen-specific antibodies in the response; and/or
2) A difference with respect to the immobilized binding structure (but related to the same antigenic agent). This kind of experiment will among others illustrate distribution of specificities of the antigen-specific antibodies in the response including cross-reacting determinants of various antigenic agents; and/or
3) A difference with respect to flow rate. This will assist in understanding the distribution of the affinity of the antibodies of the response. See above.

A still another kind of additional experiments utilizes samples that have been collected from the same or different hosts at different periods of times compared to the sample used in the first experiment or from the start of the immunization (if available). Typical periods of times between sample collections are in the interval from 1 day to 6 months or even up to a year. Potentially it is also of interest to make additional experiments according to steps (i)-(iv) with samples collected from a host after longer periods of time in order to study how a humoral immune response will decline after the cessation of the actual immunization protocol. This kind of additional experiments may be combined with experiments according to one or more of additional experiments (1)-(3) above.

A still another kind of additional experiments utilize analyte samples that differ with respect to the individual from which the samples have been obtained, for instance the same kind of sample from different individuals of the same or different species.

The additional experiments discussed above may be carried out in separate microchannel structures on the same microfluidic device and/or in different microfluidic devices. The number of each of these additional experiments may be one, two, three or more. Where appropriate the experiments are performed in parallel, for instance in different microchannel structures of the same device.

In total the invention will be a valuable tool for monitoring an immune response as a function of affinity, specificity, kind of antibodies, and stage in the immunization.

The first aspect of the invention and the additional independent aspect discussed above may be used in the clinical diagnosis. Interesting diseases are autoimmune diseases, IgE mediated allergies, parasitic infestations, infectious diseases including infections by bacteria, viruses, fungi, moulds and any other disease for which an antigen-specific antibody assay may be used in the clinical diagnosis of an individual. Either the total amount of anti-BS antibodies or anti-BS antibodies classified as high affinity antibodies are then measured and used as an indicator of the disease as such or of the severity of the disease. A distribution of captured antibodies towards the inlet end (upper section), preferably in the form of a distinct peak as discussed above, will then be indicative of higher levels and/or higher affinities of high affinity anti-BS antibodies. For many of the diseases mentioned this will be indicative of a safer diagnosis, and/or the severeness and/or the state of the disease. A more distinct peak, for instance in terms of width and height as discussed above, is therefore likely to indicate a larger diagnostic value. This does not exclude that a distribution towards the outlet end (lower section) of the solid phase might also be useful. It may at least be of value as a negative marker indicating that a certain diseased condition is likely not to be severe or not at hand.

One can envisage that for clinical diagnostic assays, the inventive method potentially will imply an improved diagnostic specificity. The improvement is likely to be a factor that is ≥ 1.1, such as ≥ 1.5 or even more, such as ≥ 2.0 compared to performing the same assay under diffusion limiting conditions, such as under static conditions for a sufficient period of time to reach equilibrium in the capture step (corresponds to step (ii) above).

### SAMPLES

The sample is typically a liquid that derives from a body fluid of a vertebrate that contains antibodies of a humoral immune response provoked in the vertebrate. Typical vertebrates are mammals, avians, amphibians, reptiles etc. Typical mammals are whales, humans, mice, rats, guinea pig, korses, cows, pigs, dogs, cats etc. Typical avians are hens, canaries, budgerigars etc. Amongst amphibians and reptiles may be mentioned those that are used as pets or are popular in zoological gardens. Typical body fluids are blood, including serum and plasma, lachrymal fluid, saliva etc. The body fluid may be adapted to the assay protocol inside or outside the microchannel structure, for instance by removal of blood corpuscles, lipids and the like, appropriate dilution, addition of anti-coagulantia, removal of undesired proteins including other Igs etc.

### GENERAL ABOUT THE MICROFLUIDIC DEVICE

A microchannel structure **(101a-h)** of a microfluidic device comprises functional parts that permit the full protocol of an experiment to be performed within the structure. A microchannel structure **(101a-h)** of the microfluidic device thus may comprise one, two, three or more functional parts selected among: a) inlet arrangement **(102,103a-h)** comprising for instance an inlet port/inlet opening **(105a-b,107a-h),** possibly together with a volume-metering unit **(106a-h,108a-h),** b) microconduits for liquid transport, c) reaction microcavity **(104a-h);** d) mixing microcavity/unit; e) unit for separating particulate matters from liquids (may be present in the inlet arrangement), f) unit for separating dissolved or suspended components in the sample from each other, for instance by capillary electrophoresis, chromatography and the like; g) detection microcavity; h) waste conduit/microcavity **(112,115a-h);** i) valve **(109a-h,110a-h);** j) vent **(116a-i)** to ambient atmosphere; liquid split (liquid router) etc. A functional part may have more than one functionality, e.g. reaction microcavity **(104a-h)** and a detection microcavity may coincide. Various kinds of functional units in microfluidic devices have been described by Gyros AB/Amersham Pharmacia Biotech AB: WO 99055827, WO 99058245, WO 02074438, WO 02075312, WO 03018198 (US 20030044322), WO 03034598, WO 05032999, WO 04103890, PCT/SE2005/000403 and by Tecan/Gamera Biosciences: WO 01087487, WO 01087486, WO 00079285, WO 00078455, WO 00069560, WO 98007019, WO 98053311.

In advantageous forms a reaction microcavity **(104a-h)** intended for a porous bed as described above is connected to one or more inlet arrangements (upstream direction) **(102,103a-h),** each of which comprises an inlet port **(105a-b,107a-h)** and at least one volume-metering unit **(106a-h,108a-h).** In one advantageous variant, there is one separate inlet arrangement **(103ah)** per microchannel structure **(101a-h)** and reaction microcavity **(104a-h)** intended to contain the solid phase material. In another advantageous variant, the inlet arrangement **(102)** is common to all or a subset **(100)** of microchannel structures **(101a-h)** and reaction microcavities **(104a-h)** intended to contain the solid phase material, and comprises a common inlet port **(105a-b)** and a distribution manifold with one volume-metering unit **(106a-h)** for each microchannel structure/reaction microcavity **(101a-h/104a-h)** of the subset **(100).** In both variants, each of the volume-metering units **(106a-h,108a-h)** in turn is communicating with downstream parts of its microchannel structure **(101a-h),** e.g. the reaction microcavity **(104a-h).** Microchannel structures linked together by a common inlet arrangement **(102)** and/or common distribution manifold define a group or subset **(100)** of microchannel structures. Each volume-metering unit **(106a-h,108a-h)** typically has a valve **(109a-h,110a-h)** at its outlet end. This valve is typically passive, for instance utilizing a change in chemical surface characteristics at the outlet end, such as a boundary between a hydrophilic and hydrophobic surface (hydrophobic surface break) (WO 99058245 (Amersham Pharmacia Biotech AB)) and/or in geometric/physical surface characteristics (WO 98007019 (Gamera)).

Typical inlet arrangements with inlet ports, volume-metering units, distribution manifolds, valves etc have been presented in WO 02074438, WO 02075312, WO 02075775 and WO 02075776 (all Gyros AB).

The microfluidic device may also comprise other common microchannels/micro conduits connecting different microchannel structures. Common channels including their various parts such as inlet ports, outlet ports, vents, *etc*., are considered part of each of the microchannel structures they are communicating with.

Common microchannels make it possible to construe microfluidic devices in which the microchannel structures form networks. See for instance US 6,479,299 (Caliper).

Each microchannel structure has at least one inlet opening **(105a-b,107a-h)** for liquids and at least one outlet opening for excess of air (vents) **(116a-i)** and possibly also for liquids (circles in the waste channel **(112)).**

The microfluidic device may also comprise microchannel structures that have no reaction microcavity for retaining a solid phase material according to the invention.

The microfludic device contains a plurality of microchannel structures/device intended to contain the solid phase according to the invention. Plurality in this context means two, three or more microchannel structures and typically is ≥ 10, e.g. ≥ 25 or ≥ 90 or ≥ 180 or ≥ 270 or ≥ 360.

Different principles may be utilized for transporting the liquid within the microfluidic device/microchannel structures between two or more of the functional parts described above. Inertia force may be used, for instance by spinning the disc as discussed in the subsequent paragraph. Other useful forces are capillary forces, electrokinetic forces, non-electrokinetic forces such as capillary forces, hydrostatic pressure etc.

The microfluidic device typically is in the form of a disc. The preferred formats have an axis of symmetry (Cₙ) that is perpendicular to or coincides with the disc plane, where n is an integer ≥ 2, 3, 4 or 5, preferably ∞ (C_{∞}). In other words the disc may be rectangular, such as square-shaped and other polygonal forms but is preferably circular. Once the proper disc format has been selected centrifugal force may be used for driving liquid flow. Spinning the device around a spin axis that typically is perpendicular or parallel to the disc plane may create the necessary centrifugal force. In the most obvious variants at the priority date, the spin axis coincides with the above-mentioned axis of symmetry. Variants in which the spin axis is separate from an axis of symmetry and not perpendicular to a disc plane (preferably at an angle of 0°) are given in WO 04050247 (Gyros AB).

For preferred centrifugal-based variants, each microchannel structure comprises one upstream section that is at a shorter radial distance than a downstream section (from the spin axis). The reaction microcavity intended for the porous bed is typically at a radial position intermediary to the two sections.

If centrifugal force is used for driving liquid flow through the reaction microcavity/solid phase, the reaction microcavity is typically oriented with the flow direction essentially radially outwards from the spin axis.

The preferred devices are typically disc-shaped with sizes and/or forms similar to the conventional CD-format, e.g. sizes that are in the interval from 10% up to 300 % of a circular disc with the conventional CD-radii (12 cm). The upper and/or lower sides of the disc may or may not be planar.

Microchannels/microcavities of a microfluidic devices may be manufactured from an essentially planar substrate surface that exhibits the channels/cavities in uncovered form that in a subsequent step are covered by another essentially planar substrate (lid). See WO 91016966 (Pharmacia Biotech AB) and WO 01054810 (Gyros AB). Both substrates are preferably fabricated from plastic material, e.g. plastic polymeric material.

The fouling activity and hydrophilicity of inner surfaces should be balanced in relation to the application. See for instance WO 0147637 (Gyros AB).

Introduction of surface coats on interior surfaces of a microfluidic device in a production mode preferably will take place as discussed in SE 04000007-1 and US 60/534,832 (both Gyros AB).

The terms "wettable" (hydrophilic) and "non-wettable" (hydrophobic) contemplate that a surface has a water contact angle ≤ 90° or ≥ 90°, respectively. In order to facilitate efficient transport of a liquid between different functional parts, inner surfaces of the individual parts should primarily be wettable, preferably with a contact angle ≤ 60° such as ≤ 50° or ≤ 40° or ≤ 30° or ≤ 20°. These wettability values apply for at least one, two, three or four of the inner walls of a microconduit. In case one or more of the inner walls have a higher water contact angle this can be compensated for by a lower water contact angle for the inner wall(s). The wettability, in particular in inlet arrangements should be adapted such that an aqueous liquid will be able to fill up an intended microcavity/microconduit by capillarity (self suction) once the liquid has started to enter the cavity/microconduit. A hydrophilic inner surface in a microchannel structure may comprise one or more local hydrophobic surface breaks in a hydrophilic inner wall, for instance for introducing a passive valve, an anti-wicking means, a vent solely function as a vent to ambient atmosphere etc (rectangles in figure 1). See for instance WO 99058245, WO 02074438, WO 04103891 (US SN 10/849321), and WO 04 103890 (all Gyros AB).

Contact angles refer to values at the temperature of use, typically +25°C, are static and can be measured by the method illustrated in WO 00056808 and WO 01047637 (all Gyros AB).

### EXPERIMENTAL PART

### ANTIBODY RESPONSE UPON VACCINATION.

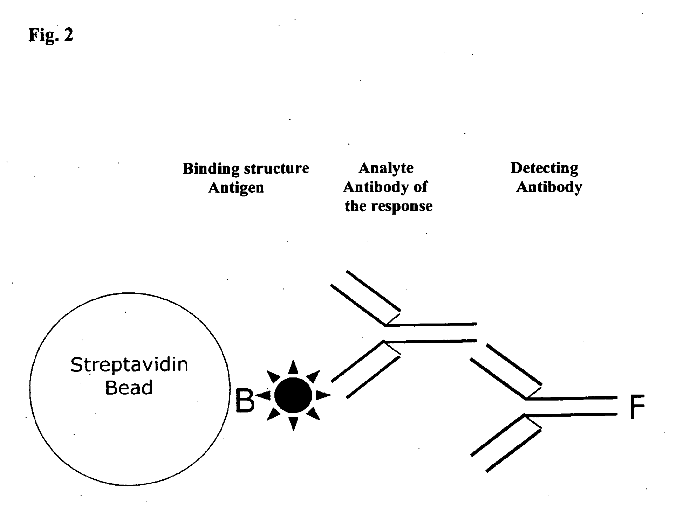

**Assay**: A preparation of biotinylated Porcine Parvo Virus (PPV) is attached to pre-formed streptavidin beads in a circular microfluidic disc. Appropriate dilutions of serum are contacted with immobilized virus antigen under flow conditions. High affinity antibodies (analyte) are captured during sample passage through the column whereas other immunoglobulin molecules pass the column or are subsequently washed out of the column. Finally a fluorophor (F, ALEXA)-labelled antibody (Ab) directed against mouse IgG subclasses is used for quantification of bound immunoglobulins. Everything is as outlined in the preferred variants given in the experimental part of WO 04083108 and WO 04083109 (all of Gyros AB) except that the biotinylated antibody now is replaced with the antigen Porcine Parvo Virus (antigenic agent, binding structure). The instrument was a Gyrolab Workstation equipped with laser fluorescence detector and the microfluidic disc a Bioaffy CD microlaboratory, both being products of Gyros AB, Uppsala, Sweden. The formed complex is schematically given in figure 2.

Indications have been obtained that there may be advantages in placing a dummy bed of particles in front of the particle bed exposing the PPV antigen. Most likely it will be simplest if the dummy bed contains the same kind of dextran coated particles as the PPV-bed. See under "Step (i): Reaction microcavity (104a-h), the immobilized binding structure, and the solid phase material" above.

**Vaccination protocol:** Mice were vaccinated with Porcine Parvo Virus in appropriate adjuvants. Serum were collected 1 and 3 weeks after a booster vaccination and analysed for presence of antigen specific antibodies directed against Porcine Parvo Virus antigen belonging to subclasses IgG1 and IgG2a, respectively.

## Claims

1. A method for grading a polyclonal antibody response with respect to a desired binding specificity, said response being provoked by an antigenic agent, **characterized in** comprising the steps of:
(i) providing a reaction microcavity having an inlet and a outlet end and containing a solid phase that exposes an immobilized binding structure (BS) that is capable of affinity binding antibodies (Ab) of the response,
(ii) flowing a liquid sample containing antibodies of the response in the direction from the inlet end to the outlet end through the solid phase,
(iii) determining the distribution of antibodies, which are captured during step (ii), along the flow direction of the solid phase, and
(iv) grading the response based on the distribution, determined in step (iii).

2. The method of claim 1, **characterized in that** the grading is according to presence or absence of high-affinity antibodies.

3. The method according to any of claims 1-2, **characterized in that** the sample is an Ig-containing sample deriving from a mammalian individual or a corresponding sample from another vertebrate.

4. The method according to any of claims 1-3, **characterized in that** steps (i)-(iv) are repeated in a further experiment with a sample that is taken from the same host after the sample of the 1^{st} experiment is taken.

5. The method according to any of claims 1-4, **characterized in that** steps (i)-(iv) are repeated in a further experiment that utilizes a detectable affinity reactant in step (iii) for determining the distribution in step (iii), said affinity reactant differs with respect to class or subclass specificity compared to the corresponding affinity reactant in the 1^{st} experiment.

6. The method according to any of claims 1-5, **characterized in that** steps (i)-(iv) are repeated in a further experiment that utilizes an immobilized binding structure (second BS) that is different from the immobilized binding structure (first BS) that is utilized in the 1^{st} experiment, first and second BS being related to the same antigenic agent.

7. The method according to any of claims 1-6, **characterized in that** steps (i)-(iv) are repeated in a further experiment with a flow rate that differs from the 1^{st} experiment.

8. The method according to any of the claim 4-7, **characterized in that** said 1^{st} and at least on of said further experiments, whichever are carried out in the method, are carried out in parallel.

9. The method according to claim 8, wherein said 1^{st} and at least one of said further experiments are carried out in the same microfluidic device.

## Patentansprüche

1. Verfahren zum Klassifizieren einer polyclonaler Antikörper-Antwort hinsichtlich einer gewünschten Bindungsspezifität, wobei die Antwort durch ein antigenes Agens hervorgerufen wird, **dadurch gekennzeichnet, dass** das Verfahren die folgenden Schritte umfasst:
(i) Bereitstellen einer Reaktions-Mikrokavität, die ein Einlass- und ein Auslass-Ende hat und eine Festphase enthält, die eine immobilisierte Bindungsstruktur (BS) exponiert, die zum Affinitätsbinden von Antikörpern (Ab) der Antwort in der Lage ist,
(ii) Fließenlassen einer flüssigen Probe, enthaltend Antikörper der Antwort, in Richtung vom Einlass-Ende zum Auslass-Ende durch die Festphase,
(iii) Bestimmen der Verteilung der Antikörper, die während Schritt (ii) eingefangen werden, entlang der Fließrichtung der Festphase, und
(iv) Klassifizieren der Antwort basierend auf der in Schritt (iii) bestimmten Verteilung.

2. Verfahren nach Anspruch 1, **dadurch gekennzeichnet, dass** das Klassifizieren nach Anwesenheit oder Abwesenheit hochaffiner Antikörper erfolgt.

3. Verfahren nach einem der Ansprüche 1-2, **dadurch gekennzeichnet, dass** die Probe eine Ig-enthaltende Probe ist, die von einem Säuger-Individuum oder einer entsprechenden Probe von einem anderen Wirbeltier abgeleitet ist.

4. Verfahren nach einem der Ansprüche 1-3, **dadurch gekennzeichnet, dass** Schritte (i)-(iv) in einem weiteren Experiment mit einer Probe wiederholt werden, die vom selben Wirt entnommen worden ist, nachdem die Probe des ersten Experiments entnommen worden ist.

5. Verfahren nach einem der Ansprüche 1-4, **dadurch gekennzeichnet, dass** Schritte (i)-(iv) in einem weiteren Experiment wiederholt werden, das einen nachweisbaren Affinitäts-Reaktanden in Schritt (iii) für das Bestimmen der Verteilung in Schritt (iii) verwendet, wobei der Affinitäts-Reaktand hinsichtlich der Klassen- oder Subklassenspezifität im Vergleich zum entsprechenden Affinitäts-Reaktanden im ersten Experiment abweicht.

6. Verfahren nach einem der Ansprüche 1-5, **dadurch gekennzeichnet, dass** Schritte (i)-(iv) in einem weiteren Experiment wiederholt werden, das eine immobilisierte Bindungsstruktur (zweite BS) verwendet, die von der immobilisierten Bindungsstruktur (erste BS), die im ersten Experiment verwendet wird, verschieden ist, wobei sich die erste und zweite BS auf das gleiche antigene Agens beziehen.

7. Verfahren nach einem der Ansprüche 1-6, **dadurch gekennzeichnet, dass** die Schritte (i)-(iv) in einem weiteren Experiment mit einer Flussrate wiederholt werden, die vom ersten Experiment abweicht.

8. Verfahren nach einem der Ansprüche 4-7, **dadurch gekennzeichnet, dass** das erste und mindestens eines der weiteren Experimente, welche auch immer in dem Verfahren ausgeführt werden, parallel ausgeführt werden.

9. Verfahren nach Anspruch 8, wobei das erste und mindestens eines der weiteren Experimente in der gleichen Mikrofluid-Vorrichtung ausgeführt werden.

## Revendications

1. Procédé pour évaluer une réponse d'anticorps polyclonaux vis-à-vis d'une spécificité de liaison souhaitée, ladite réponse étant provoquée par un agent antigénique, **caractérisé en ce qu'**il comprend les étapes consistant :
(i) à fournir une microcavité de réaction ayant une extrémité d'entrée et une extrémité de sortie et contenant une phase solide qui expose une structure de liaison (BS) immobilisée qui est capable de se lier par affinité à des anticorps (Ab) de la réponse,
(ii) à faire s'écouler un échantillon liquide contenant des anticorps de la réponse dans la direction allant de l'extrémité d'entrée jusqu'à l'extrémité de sortie, à travers la phase solide,
(iii) à déterminer la distribution des anticorps, qui sont capturés pendant l'étape (ii), le long de la direction d'écoulement de la phase solide, et
(iv) à évaluer la réponse en fonction de la distribution déterminée dans l'étape (iii).

2. Procédé selon la revendication 1, **caractérisé en ce que** l'évaluation est fonction de la présence ou de l'absence d'anticorps de haute affinité.

3. Procédé selon l'une quelconque des revendications 1-2, **caractérisé en ce que** l'échantillon est un échantillon contenant une Ig et provenant d'un individu mammifère ou un échantillon correspondant d'un autre vertébré.

4. Procédé selon l'une quelconque des revendications 1-3, **caractérisé en ce que** les étapes (i) à (iv) sont répétées dans une expérience supplémentaire avec un échantillon qui est prélevé auprès du même hôte après avoir prélevé l'échantillon de la 1^{ère} expérience.

5. Procédé selon l'une quelconque des revendications 1-4, **caractérisé en ce que** les étapes (i) à (iv) sont répétées dans une expérience supplémentaire qui utilise un réactif d'affinité détectable dans l'étape (iii) pour déterminer la distribution dans l'étape (iii), ledit réactif d'affinité différant quant à la spécificité pour une classe ou sous-classe par rapport au réactif d'affinité correspondant dans la 1^{ère} expérience.

6. Procédé selon l'une quelconque des revendications 1-5, **caractérisé en ce que** les étapes (i) à (iv) sont répétées dans une expérience supplémentaire qui utilise une structure de liaison (seconde BS) immobilisée qui est différente de la structure de liaison (première BS) immobilisée qui est utilisée dans la 1^{ère} expérience, les première et seconde BS se rapportant au même agent antigénique.

7. Procédé selon l'une quelconque des revendications 1-6, **caractérisé en ce que** les étapes (i) à (iv) sont répétées dans une expérience supplémentaire avec un débit qui diffère de la 1^{ère} expérience.

8. Procédé selon l'une quelconque des revendications 4-7, **caractérisé en ce que** ladite 1^{ère} et au moins l'une desdites expériences supplémentaires, qui sont effectuées dans le procédé, sont effectuées en parallèle.

9. Procédé selon la revendication 8, dans lequel ladite 1^{ère} et au moins l'une desdites expériences supplémentaires sont effectuées dans le même dispositif microfluidique.
